Europäisches Patentamt

European Patent Office

Office européen des brevets

(19)

(11) **EP 0 630 401 B1**

(12) **EUROPÄISCHE PATENTSCHRIFT**

(45) Veröffentlichungstag und Bekanntmachung des Hinweises auf die Patenterteilung:
**15.10.1997 Patentblatt 1997/42**

(51) Int Cl.$^6$: **C12N 1/20**, C12N 9/52
// (C12N1/20, C12R1:01)

(21) Anmeldenummer: **93907830.9**

(22) Anmeldetag: **12.03.1993**

(86) Internationale Anmeldenummer:
**PCT/EP93/00569**

(87) Internationale Veröffentlichungsnummer:
**WO 93/18134 (16.09.1993 Gazette 1993/22)**

(54) **MIKROORGANISMUS STAMM W, VON DIESEM ERHÄLTLICHE ENZYMZUSAMMENSETZUNG, VERWENDUNG DES MIKROORGANISMUS UND VERFAHREN ZUR HYDROLYSE INSBESONDERE VON KERATIN**

STRAIN W MICRO-ORGANISM, ENZYME COMPOSITIONS OBTAINABLE THEREFROM, USE OF THE MICRO-ORGANISM AND HYDROLYSIS PROCESS ESPECIALLY OF KERATIN

MICROORGANISME, SOUCHE W, COMPOSITION ENZYMATIQUE QU'IL PERMET D'OBTENIR, UTILISATION DU MICRO-ORGANISME ET PROCEDE D'HYDROLYSE, NOTAMMENT DE LA KERATINE

(84) Benannte Vertragsstaaten:
**AT BE CH DE DK ES FR GB GR IE IT LI LU MC NL PT SE**

(30) Priorität: **13.03.1992 DE 4208275**

(43) Veröffentlichungstag der Anmeldung:
**28.12.1994 Patentblatt 1994/52**

(73) Patentinhaber: **DEGUSSA AG**
**60311 Frankfurt (DE)**

(72) Erfinder: **ANTRANIKIAN, Garabed**
**D-2105 Seevetal 1 (DE)**

(56) Entgegenhaltungen:
**FR-A- 2 590 450**

- **DATABASE WPIL Section Ch, Week 9131, Derwent Publications Ltd., London, GB; Class D16, AN 91-225306**

**Beschreibung**

Technisches Gebiet der Erfindung

Die Erfindung betrifft einen neuen Mikroorganismus mit proteolytischen Eigenschaften, eine von dem Mikroorganismus erhältliche Enzymzusammensetzung, die Verwendung des Mikroorganismus sowie Verfahren zur Hydrolyse insbesondere von Keratin.

Beschreibung des Stands der Technik

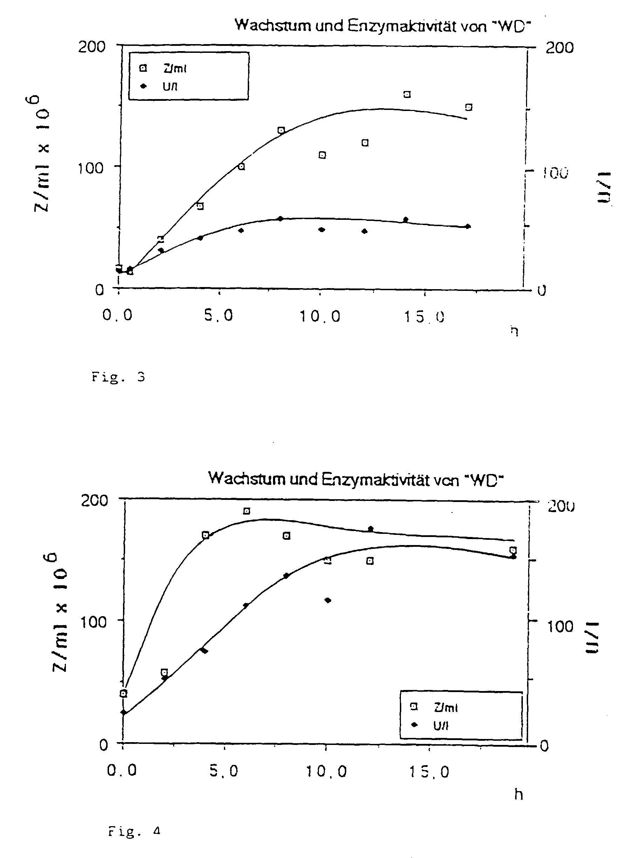

Es ist bekannt, daß spezielle Enzyme, die Proteasen, Proteine hydrolysieren, wobei Oligopeptide und schließlich Aminosäuren entstehen. Diese Enzyme werden auch von Mikroorganismen gebildet, wie z. B. von Bakterien der Gattung Pseudomonas, Bacillus oder Proteus. Einige Enzyme, die sogenannten thermophilen Enzyme, zeigen die o. g. Aktivität auch bei Temperaturen ≥ 70°C und ermöglichen hierdurch insbesondere industriell interessante Hydrolysen. Eine Quelle für solche thermophilen Enzyme sind z. B. thermophile Mikroorganismen. Einen Überblick hierüber gibt BFE 9, No. 7/8, S. 466 - 470 (1992) in dem Artikel "Thermophilic Enzymes as Industrial Catalysts?" von K. Peek et al. Ein weiteres Enzym, das oberhalb 70°C noch eine gewisse Aktivität zeigt, ist aus Biosci. Biotech. Biochem., 56 (10), S. 1667 - 9 (1992) bekannt. Dieses Enzym zeigt neben gewöhnlichen proteolytischen Eigenschaften auch eine gewisse Keratinase-Aktivität. So wurden mit diesem Enzym zwar Haare bei einer Temperatur bis zu 90°C angegriffen, dies jedoch bei einem pH von 11,0 und über den Zeitraum von 1 h, obwohl das Enzym unter den angegebenen Bedingungen nach bereits 10 min die Hälfte seiner Aktivität eingebüßt hat (vgl. Appl. Microbiol. Biotechnol. (1989) 30, S. 120 - 124). Da die in Biosci. in Fig. 2b wiedergegebene Zersetzung von menschlichem Haar selbst bei Temperaturen oberhalb 80°C sogar noch eine höhere Zersetzung ausweist, muß diese Zersetzung wegen der Enzymdesaktivierung chemischer und nicht enzymatischer Natur sein. Aufgrund der bei 70°C stark abfallenden Stabilität dieses beschriebenen Enzyms ist dieses nicht geeignet. Keratin, wie es z. B. in Federn, Horn oder Haaren vorkommt, im industriellen Maßstab, d. h. innerhalb einiger Stunden bis wenige Tage, abzubauen, da für einen vollständig enzymatischen Abbau nur solche Keratinmengen zugesetzt werden können, die in weniger als 3 h, insbesondere deutlich schneller als in 1 h, abgebaut sind.

Darstellung der Erfindung

Aufgabe der vorliegenden Erfindung ist das Isolieren und Bereitstellen eines Mikroorganismus, der über proteolytische Eigenschaften verfügt und auch Keratin in Naturprodukten in verhältnismäßig kurzer Zeit abbauen kann. Aufgabe der vorliegenden Erfindung ist daher auch eine von diesem Mikroorganismus erhältliche keratinabbauende Enzymzusammensetzung und ein Verfahren zum Abbau insbesondere von Keratin.

Hinsichtlich des Mikroorganismus wird die gestellte Aufgabe durch den Gegenstand des Anspruchs 1 gelöst.

Der Mikroorganismus, Stamm W, wurde bei der Deutschen Sammlung für Mikroorganismen und Zellkulturen GmbH, Mascheroder Weg 1b, D-3300 Braunschweig, am 12.03.1992 hinterlegt und hat die Eingangsnummer DSM 7003 erhalten. Aufgrund seiner Eigenschaften hat der Mikroorganismus die Bezeichnung Fervidobacterium pennavorans erhalten.

Der Mikroorganismus ist lebensfähig und gemäß dem Budapester Vertrag über die internationale Anerkennung der Hinterlegung von Mikroorganismen für die Zwecke von Patentverfahren hinterlegt und freigegeben. Entsprechende Unterlagen, Erklärungen und Formblatt PCT/RO/134 sind der Anmeldung beigefügt.

Der Mikroorganismus wie auch die von diesem Mikroorganismus gebildeten Proteasen verfügen über eine gute Aktivität, so daß auch eine Enzymzusammensetzung, die aus dem Mikroorganismus erhältlich ist, eine gute in vitro-Aktivität gegenüber Proteinen, insbesondere gegenüber Keratin bzw. keratinhaltigen Substanzen, verfügt. Somit ist auch diese Enzymzusammensetzung isoliert von dem Mikroorganismus einsetzbar.

F. pennavorans ist der erste bekannte thermophile Mikroorganismus, der Federn (z. B. Hühnerfedern) als Substrat verwertet, und das sehr gut.

Sowohl mit dem neuen Mikroorganismus als auch mit der Enzymzusammensetzung lassen sich nicht nur unlösliche Strukturproteine, sondern auch lösliche Proteine behandeln. Der Abbau kann vollständig erfolgen, i. d. R. entstehen jedoch neben einzelnen Aminosäuren auch kurze Peptidsequenzen, die gewünschtenfalls auch industriell weiterverwertet werden können. Gegenüber herkömmlichen Verfahren hat der biologische Abbau vor allem den Vorteil, daß auf eine chemische und/oder mechanische Behandlung der Proteine verzichtet werden kann. Ein weiterer Vorteil bei der Anwendung des neuen Mikroorganismus bzw. der Enzymzusammensetzung besteht darin, daß infolge der thermischen Stabilität der Enzyme die hydrolytische Spaltung der peptidischen Bindung günstig bei hoher Temperatur erfolgen kann, und daß der biologische Prozeß hierdurch insgesamt kontaminationssicherer wird, so daß vor allem auch einer Kontamination mit patogenen Mikroorganismen entgegengewirkt werden kann. Da die beim proteolytischen Abbau erhaltenen Oligopeptid bzw. einzelnen Aminosäuren ein hervorragendes Substrat für viele Mikroorganismen darstellen, ist die erfindungsgemäß mögliche Verfahrensweise insbesondere für die industrielle Anwendung sehr vorteilhaft. Durch die höhere Temperatur erhält man auch eine bessere Substratlöslichkeit und einen leichteren Angriff des Enzyms am Substrat. Außerdem ist die erfindungsgemäße Enzymzusammensetzung auch sonst sehr stabil, z. B. gegen SDS und Harnstoff.

Neben der bevorzugten Zersetzung von keratinhaltigen Substanzen kann der neue Mikroorganismus bzw. die Enzymzusammensetzung z. B. auch zur Beseitigung proteinhaltiger Abfälle eingesetzt werden, wobei sich in Kombination mit anderen Mikroorganismen, wie z. B. methanogenen Bakterien, weitere Vorteile ergeben. Werden die gebildeten Peptide oder Aminosäuren weiter zu Methan umgesetzt, kann gleichzeitig eine Energieeinsparung erfolgen. Darüber hinaus können auch z. B. Alkohole oder andere Säuren gebildet werden.

Die zur Erfindung gehörige Enzymzusammensetzung ist erhältlich aus einer den Mikroorganismus Stamm W enthaltenden Kultur, wobei der Kulturüberstand abgetrennt und ggf. konzentriert wird. Ebenso ist eine Enzymzusammensetzung aus einer den Mikroorganismus Stamm W enthaltenden Kultur erhältlich, wobei die Zellen aufgeschlossen werden. Zur Gewinnung der Enzymzusammensetzung der genannten Art ist es vorteilhaft, wenn der Mikroorganismus Stamm W zur Enzymproduktion bzw. zur Enzyminduktion in einem Tryptonmedium kultiviert wird.

Zur Erfindung gehört auch eine Enzymzusammensetzung, die insbesondere wie oben beschrieben zugänglich ist, wobei die Enzymzusammensetzung ein oder mehrere Enzyme beinhalten kann und Proteaseaktivität besitzt, und wobei

- das Temperaturoptimum (bei pH 10) im Bereich von 70 bis 90°C, insbesondere bei 80°C liegt;

- das pH-Optimum (bei 80°C) im Bereich von pH 7 bis pH 11, insbesondere im Bereich von pH 9 bis pH 10 liegt;

- Gelfiltration ein proteolytisches Enzym oder Enzymkomplex mit einem Molekulargewicht von ca. 200 000 Dalton und darüber ausweist und eine Molekulargewichtsbestimmung mit einem SDS-Aktivitätsgel zwei Banden im Bereich zwischen ca. 200.000 und ca. 330.000 zeigt;

- der $E_{405}$-Wert (gegen Blindwert) nach 17 h Inkubation bei 60°C, 0,2 mM Substrat in Phosphatpuffer pH 7 und 0.3 mg/ml Enzymrohextrakt, Meßstrecke 1 cm, bei N-Succinyl-Phe-pNA, N-Succinyl-Ala-Ala-Pro-Phe-pNA und Z-Arg-pNA > 0,5, bei H-Gly-Glu-pNA zwischen 0,3 und 0.5, bei Acetyl-Ala-pNA zwischen 0,1 und 0,3, und bei Benzoyl-DL-Arg-pNA, Z-Gly-Pro-pNA. Benzoyl-Lys-pNA, Z-Arg-pNA und Acetyl-Tyr-pNA unter 0,1 liegt;

- die folgenden Inhibitoren bei den angegebenen Konzentrationen etwa die folgenden Restaktivitäten ergeben:

| Inhibitor | Restakt. (1 mM) | Restakt. (5 mM) |
|---|---|---|
| ohne | 100 % | 100 % |
| Pefabloc SC (Ser.) | 83 % | - |
| PMSF (Ser.) | 14 % | 10 % |
| EDTA (Metallo.) | 93 % | 85 % |
| Iodoacetat (Cyst.) | 83 % | 76 % |

- die Thermostabilitäten wie folgt sind:

- bei pH 7 und 8,5 über 20 h bei 70°C und 80°C stabil,
- bei 90°C nach 6 h weniger als 10 % Restaktivität,

- bei pH 10, 80°C und 90°C nach 15 min Totalverlust der Aktivität
- bei 70°C etwa 40 % Restaktivität nach 6 h, nach 20 h Totalverlust.

Die Bezeichnung "Enzymzusammensetzung", die für ein oder mehrere Enzyme stehen kann, wurde gewählt, da der proteolytisch aktive Extrakt ein sehr hohes Molekulargewicht hat (ca. 200 000 bei Gelfiltration auf Superdex 200 und zwischen ca. 200 000 und ca. 330 000 bei SDS-PAGE (sodium dodecylsulfatpolyacrylamid gradientgel slab electrophoresis) auf amido black stained gel mit 0,1 % Gelantine als Substrat). Dies deutet auf die mögliche Existenz eines Enzymkomplexes hin, der möglicherweise, unter den beschriebenen Bedingungen jedoch nicht auftrennbar, gleiche oder unterschiedliche Untereinheiten enthält.

Prinzipiell lassen sich die erfindungsgemäßen Enzymzusammensetzungen sowie die erfindungsgemäßen Verfahren mit weiteren Enzymen bzw. -zusammensetzungen - bei entsprechender Stabilität - unmittelbar, ansonsten vor- oder nachgeschaltet kombinieren. Hierdurch kann z. B. der Abbau von Oligopeptiden, die erfindungsgemäß durch Abbau größerer Peptide, insbesondere unlöslicher Peptide, entstehen, zu Aminosäuren gefördert werden.

Der Mikroorganismus Stamm W und/oder die beschriebenen Enzymzusammensetzungen finden Verwendung zum Abbau von Proteinen wie insbesondere auch Keratin bzw. von solchen Stoffen, die diese Proteine beinhalten, insbesondere von Federn, Horn oder Haaren. Besonders günstig ist dabei der Abbau von Federn, da gerade dieser Abbau aufgrund des besonderen Aufbaus von Federn und deren Zusammensetzung bislang enzymatisch besondere Schwierigkeiten bereitete.

Da von Hühnerfarmen mehr als 10 000 t/a Federabfall produziert wird, und Federn zu ca. 95 Gew.-% aus β-Keratin bestehen, bietet die Erfindung eine günstige Alternative zum rein chemischen Abbau von Federn zu Aminosäuren.

Der Mikroorganismus Stamm W findet außerdem Verwendung zur Isolierung eines eine Keratinase codierenden Gens und/oder zur Insertion eines eine Keratinase codierenden Gens in einen anderen Mikroorganismus. Hierbei handelt es sich um routinemäßig durchgeführte bzw. durchführbare Verfahren analog Winnaker E. L., Gene und Klone: Eine Einführung in die Gentechnologie, VCH, Weinheim. 1985; Maniatis, Fritsch, Sambrock: Molecular Cloning, A Laboratory Manual, Cold Spring Harbour Lab. New York, 1989, wobei das eine Keratinase codierende Gen aus dem Mikroorganismus Stamm W z. B. in ein E. coli oder Bacillus insertiert wird und der E. coli dann zur Gewinnung der Keratinase gezüchtet wird. Die Keratinase kann aufgrund ihrer Temperaturstabilität dann leicht aus diesem E. coli oder Bacillus durch Erhitzen abgetrennt werden. Da der Mikroorganismus Stamm W als anaerober Mikroorganismus nur langsam gezüchtet werden kann, erlaubt die erfindungsgemäße Insertion in den leicht zu kultivierenden E. coli oder Bacillus eine schnellere Anzucht und höhere Enzymausbeuten.

Ein zur Erfindung gehörendes Verfahren zur Hydrolyse von Peptiden, insbesondere von Keratin oder keratinhaltiger Substanz, wie Federn, Haare oder Horn, in Oligopeptide und/oder Aminosäuren bei einer Temperatur > 50°C mittels eines oder mehrerer Enzyme wird derart durchgeführt, daß die Hydrolyse bei einer Temperatur zwischen 50°C und 105°C.erfolgt, wobei der pH zwischen 4 und < 11 eingestellt wird und wobei durch die Wahl der zugegebenen Enzymmenge, insbesondere aber durch Wahl der Temperatur und des pHs Sorge getragen wird, daß der enzymatische Abbau ein mehrfaches von einem eventuell begleitenden chemischen Abbau beträgt. Besonders vorteilhaft ist dabei ein pH von höchstens 10,5. Je höher der pH liegt, desto höher ist auch der Anteil des chemischen Abbaus, wobei gleichzeitig auch die Menge an racemisierten Aminosäuren oder an Oligopeptiden mit racemisierten Aminosäuren zunimmt.

Desweiteren kann gemäß der vorliegenden Erfindung ein Verfahren zur Hydrolyse von Peptiden, insbesondere von Keratin oder keratinhaltiger Substanz, wie Federn, Haare oder Horn, in Oligopeptiden und/oder Aminosäuren mittels eines oder mehrerer Enzyme bei einer Temperatur ≥ 70°C durchgeführt werden, bei dem der enzymatische Abbau anaerob ist. Besonders günstig ist eine Übertragung des anaeroben Abbaus auf die zuerst beschriebene Verfahrensweise. Grundsätzlich läßt sich die anaerobe Verfahrensweise bei einem pH zwischen 4 und 12 durchführen, wobei wiederum der pH unter 11 und insbesondere bis 10,5 bevorzugt ist. Mit dem anaeroben Abbau lassen sich besonders saubere Aminosäuren gewinnen, insbesondere schwefelhaltige Aminosäuren, wie Cystin und Cystein, werden nicht oxidiert.

Günstig ist auch eine dritte Verfahrensvariante, bei der Peptide, insbesondere Keratin oder keratinhaltige Substanz, wie Federn, Haare oder Horn, in Oligopeptide und/oder Aminosäuren mittels eines oder mehrerer Enzyme hydrolysiert werden, wobei ein mesophiler Mikroorganismus eingesetzt wird, der wie oben beschrieben zugänglich ist und der ein Gen enthält, das eine bei ≥ 70°C aktive Protease, insbesondere Keratinase codiert, wobei der mesophile Mikroorganismus bei ≥ 70°C in wässrigem Medium mit dem Protein, insbesondere Keratin oder keratinhaltiger Substanz umgesetzt wird, oder wobei der mesophile Mikroorganismus, der das beschriebene Gen enthält, in wässrigem Medium auf ≥ 70°C erhitzt und das wässrige Medium, ggf. nach Abtrennen ausgefallener Komponenten, wie z. B. Teile des mesophilen Mikroorganismus, mit dem Protein, insbesondere Keratin oder der keratinhaltigen Substanz versetzt wird. Das die Protease, insbesondere Keratinase codierende Gen ist insbesondere ein - wie oben beschrieben - in den mesophilen Mikroorganismus insertiertes Gen. Diese Verfahrensweise ist insbesondere für die industrielle Peptidhydrolyse, insbesondere von Federn, geeignet.

Die Verfahrensweise erlaubt ein praktisch kontaminationsfreies Arbeiten, wobei die erhaltenen Aminosäuren und/ oder Oligopeptide verhältnismäßig sauber anfallen bzw. von eventuellen Verunreinigungen, wie z. B. Teilen des Mikroorganismus, einfach, z. B. durch Filtration, abgetrennt werden können.

Gemäß der vorliegenden Erfindung ist ein weiteres Verfahren zur Hydrolyse speziell unlöslicher Proteine, wie Keratin oder keratinhaltiger Substanz, wie Federn, Haare oder Horn, in Oligopeptide und/oder Aminosäuren bei einer Temperatur > 50°C und mittels eines oder mehrerer Enzyme, vorteilhaft, wobei die Hydrolyse mit einer solchen Enzymmenge, bei einem solchen pH und einer solchen Temperatur durchgeführt wird, daß ein zugegebenes unlösliches Substrat, insbesondere Federn, Haare oder Horn, frühestens nach 3 h aufgelöst ist, wobei besonders vorteilhaft dieses Substrat nach 24 h zumindest zu 50 Gew.-% aufgelöst ist. Vorteilhaft wird dieses Verfahren nach 1 bis 4 Tagen abgebrochen, wobei dann insbesondere mindestens 80 Gew.-%, vorzugsweise über 90 Gew.-% und besonders günstig praktisch das gesamte Substrat aufgelöst ist.

Wenn bei den vorgenannten Verfahren Keratin oder keratinhaltige Substanz, insbesondere Federn, eingesetzt werden, kann vorteilhaft auf eine Vorbehandlung, wie z. B. ein Autoklavieren, insbesondere auf ein Behandeln bei einer Temperatur ≥ 130°C und besonders vorteilhaft auch auf eine Vorbehandlung ≥ 120°C, verzichtet werden. Solche Vorbehandlungen sind bei der Hydrolyse von Federn bislang üblich gewesen, hierbei wird jedoch ein beträchtlicher Anteil der Aminosäuren racemisiert.

Erfindungsgemäß können die vorgenannten Verfahren auch zweistufig durchgeführt werden, d. h. nach Auflösen zumindest eines Teils des eingesetzten Substrats, insbesondere nach Auflösen von mehr als 50 Gew.-% des eingesetzten Substrats, wird das Hydrolysat, ggf. unter anderen Bedingungen (Temperatur, pH), mit einer oder mehreren anderen Peptidasen weiterbehandelt. Dies dient der schnelleren oder überhaupt weitergehenden Hydrolyse entstandener Oligopeptide.

Mit zur Erfindung gehört auch ein Verfahren zur enzymatischen Hydrolyse von Proteinen, insbesondere von Keratin bzw. von solchen Stoffen, die diese Proteine beinhalten, insbesondere von Federn, Horn oder Haaren, wobei die Hydrolyse mit einer der ober beschriebenen Enzymzusammensetzungen und/oder mit dem Mikroorganismus Stamm W durchgeführt wird. Grundsätzlich sind die oben beschriebenen Enzymzusammensetzungen in allen erfindungsgemäßen Verfahren einsetzbar.

**Kurze Beschreibung der Zeichnungen**

Im folgenden wird die Erfindung anhand von Beispielen und Figuren näher erläutert.
Es zeigen:

Fig. 1     das pH-Optimum einer erfindungsgemäßen Enzymzusammensetzung;

Fig. 2     das Temperatur-Optimum der Enzymzusammensetzung gemäß Fig. 1;

Fig.     3 die Enzymaktivität dieser Enzymzusammensetzung;

Fig.     4 die Enzymaktivität analog Fig. 3 unter Zusatz von Trypton;

Fig. 5     den enzymatischen Abbau löslicher Substrate;

Fig. 6     den enzymatischen Abbau unlöslicher Substrate und

Fig. 7     den Abbau von Federn innerhalb von 2 Tagen.

Ausführliche Beschreibung / Wege zur Ausführung der Erfindung

**Stammcharakterisierung**

Der neue Mikroorganismus Stamm W wurde aus einer heißen Quelle der Azoren isoliert. Er läßt sich unter Stickstoff bei 70°C kultivieren. Hierzu lassen sich Hungate-Röhrchen verwenden, die mit einem Medium folgender Zusammensetzung befüllt werden:

| | | |
|---|---|---|
| $K_2HPO_4$ | 1,6 | g |
| $NaH_2PO_4$ | 1,0 | g |
| $(NH_4)_2SO_4$ | 1,5 | g |
| $NaCl$ | 3,0 | g |
| $CaCl_2 \times 2\ H_2O$ | 0,1 | g |
| $MgSO_4 \times 7\ H_2O$ | 0,3 | g |
| $FeCl_2 \times 6\ H_2O$ | 6,0 | mg |
| Trypton | 1,0. | g |
| Hefeextrakt | 1,0 | g |

| | | |
|---|---|---|
| Spurenelemente | 1,0 | ml |
| Vitamine | 1,0 | ml |
| Resazurin (1 mg/ml) | 1,0 | ml |
| $NaHCO_3$ | 1,0 | g |
| Cystein HCl | 0,5 | g |
| aqua dest ad | 1,0 | l |

pH (mit 6 N HCl) 6,0 (nach dem Autoklavieren etwa 6,3)

| Spurenelemente: | |
|---|---|
| $MnCl_2 \times 4\ H_2O$ | 1,0 g |
| $CoCl_2 \times 6\ H_2O$ | 1,0 g |
| $NiCl_2 \times 6\ H_2O$ | 0,5 g |
| $ZnCl_2$ | 0,5 g |
| $CuSO_4$ | 0,5 g |
| $H_3BO_4$ | 0,2 g |
| $Na_2MoO_4 \times 2\ H_2O$ | 0,1 g |
| $Na_2SeO_3 \times 5\ H_2O$ | 0,1 g |
| $VOSO_4 \times 5\ H_2O$ | 0,03 g |
| aqua dest ad | 1,0 l |

Vitaminlösung:

| | | |
|---|---|---|
| Biotin | 0,2 | g |
| Folsäure | 0,2 | g |
| Pyridoxin/HCl | 1,0 | g |
| Thiamin/HCl | 0,5 | g |
| Riboflavin | 0,5 | g |
| Nicotinsäure | 0,5 | g |
| DL-Calciumpanthothenat | 0,5 | g |

| | | |
|---|---|---|
| Vitamin $B_{12}$ | 0,01 | g |
| p-Aminobenzoat | 0,5 | g |
| Liponsäure | 0,5 | g |
| aqua dest ad | 1,0 | l |
| pH mit 1 N NaOH 7,0 | | |

Als Substrat wird dem Medium entweder 0,2 % Trypton oder unlösliches Protein z. B. in Form einer Feder zugegeben. Die Hungate-Röhrchen werden anschließend mit Stickstoff begast. Danach erfolgt eine Sterilisation unter allgemein üblichen Bedingungen wie z. B. bei 120°C für 30 min. Werden allerdings Strukturproteine eingesetzt, wird bei niedrigeren Temperaturen z. B. bei 100°C für 60 min sterilisiert, damit die natürlichen Proteine nicht denaturieren. Die anschließende Inkubation kann bei hohen Temperaturen erfolgen, insbesondere bei 70°C. Unter anaeroben Bedingungen wachsen die Zellen in ca. 8 bis 12 h an, wobei ein Abbau der Strukturproteine nach 3 Tagen festgestellt werden kann.

Eine Sporenbildung konnte bei Anzucht auf Sporenmedium mit und ohne Glucose nicht nachgewiesen werden. Über einen Peroxidasetest war keine Katalase nachweisbar.

Darüber hinaus weist der neue Mikroorganismus Stamm W folgende Abbaueigenschaften auf:

| Substrat | Abbau |
|---|---|
| Lactose | - |
| Glucose | - |
| Fructose | -* |

```
       Maltose                          +

       Xylose                           +

       Amylase                          +

       Arabinose                        -

       Inulin                           -

       Tween  80                        -

       HEC  (Cellulose)                 -

       Pullulan                         +

       Glycogen                         +

       Stärke                           +

       Gelantine                        ~
                                         *
       Pepton                           -
                                          *
       Bactopepton                      -

       Collagen                         -
                                         *
       Casein                           -

       Trypton                          +

       Amylopectin                      -

       Succinat                         -

       Hefeextrakt                      +
                                                    *
       Grameigenschaft        gramnegativ

       KOH-Test               keine  Reaktion

       Xylanaleban                      -
```

```
       *
         nicht  eindeutig
```

Eine Bestimmung des Guanosin+Cytosin-Gehalts der DNA mittels HPLC ergab durchschnittlich G + C = 40,0 ± 0,6 mol-% (drei Bestimmungen). Die Bestimmung erfolgte analog wie in Anal. Biochem. 81, (1977), 461 - 6 für die DNA-Isolierung; Int. J. Syst. Bact. 39, (1989), 159 - 167 für den DNA-Abbau, Referenz-DNA sowie G+C-Bestimmung und FEMS Microbiology Letters 25, (1984), 125 - 8 für die chromatographischen Bedingungen beschrieben.

Analytische Säule NUCLEOSIL 100-5C18 (250 × 4 mm) mit Vorsäule NUCLEOSIL 100-5C18 (20 × 4 mm); Temperatur 26°C, Laufmittel 0,6 M $(NH_4)H_2PO_4$) Acetonitril 80 : 6 (v/v), pH 4,4, 0,7 ml/min, Druck ca. 120 bar, Calibrierung mit nichtmethylierter LAMBDA-DNA (Sigma), G+C-Gehalt 49,858 mol-%, DNA-Abbau mit P1 Nuclease und Dephosphoxylierung mit alkalischer Phosphatase aus Kälberserum (bovine alkaline phosphatase).

Herstellung eines Rohextraktes

Zellen einer Fermenterkultur werden (ca. 1 g / 20 ml Na-Phosphat 50 mM pH 6,8-Puffer) per Ultraschall aufgeschlossen. (15 min, 50 %, Sufe 6 - 7, mittlere Beschallungsspitze, Branson Sonifier). Nach mikroskopischer Kontrolle auf gelungenen Aufschluß wird der Rohextrakt für 20 min, 4°C, 35.000 xg, zentrifugiert. Der so gewonnene Überstand wird für die Enzymtests eingesetzt.

Aktivität: etwa 0.3 - 0.8 U/ml, je nach Aufschluß.
Protein: etwa 1 - 2 mg/ml.

Bestimmung der proteolytischen Aktivität

Bestimmung der proteolytischen Aktivität gegenüber löslichen Proteinen

Das Prinzip der Messung proteolytischer Aktivität gegenüber löslichen Proteinen beruht darauf, daß durch enzymatische Aktivität aus Proteinen, wie z. B. Casein oder Rinderserumalbumin aromatische Aminosäuren freigesetzt werden. Diese lassen sich photometrisch nach Abstoppen der Reaktion und Ausfällen des nichtgespaltenen Proteins mit Trichloressigsäure im Überstand bei 280 nm detektieren.

Reaktionsansatz:

0,45 ml 0,25 %ige (w/v) Caseinlösung (Hammersten Quality) in einem entsprechenden B & R-Puffer (Britton & Robinson, J. Chem. Soc. (1931), p. 1456. Eine Lösung enthaltend je 0,4 M $H_3PO_4$, $CH_3COOH$ und $H_3BO_3$ in Wasser wird mit 0,2 N NaOH auf den gewünschten pH eingestellt.) werden auf Eis mit 0,05 ml enzymhaltiger Probe in E-Cups pipettiert und je nach Aktivität bis zu 60 min bei der für das Enzym optimalen Temperatur (80°C) und pH-Wert (10) inkubiert. Anschließend wird der Ansatz zum Abstoppen und Ausfällen der Proteine mit 0,5 ml 10 %iger (w/v) Trichloressigsäure (TCA) versetzt, für 15 - 30 min bei Raumtemperatur inkubiert und dann für 10 min bei 13000 Upm in einer Eppendorf-Zentrifuge (Heraeus Sepatech, Osterode) zentrifugiert. Die Extinktion des klaren Überstands wird bei 280 nm in einer Quarzküvette gegen einen nichtinkubierten Leerwert gemessen.

Zur Bestimmung der Enzymaktivität wird eine Tyrosin-Eichkurve im Bereich von 0 - 1 µM/Ansatz aufgenommen. Eine Enzymaktivität (1 Unit) wird als diejenige Menge definiert, die 1 µmol aromatische Aminosäuren/min freisetzte. Aus der Steigung der Eichkurve ergibt sich folgende Berechnung:

$$U/l = \frac{\Delta E \times 1 \times 20.000}{t\ [min]}$$

Herstellung der Enzymzusammensetzung

Um die erfindungsgemäße Enzymzusammensetzung zu erhalten, werden die in einer Reinkultur oder Mischkultur angezogenen Zellen bei 10.000 x g abgetrennt, so daß sich die Enzymzusammensetzung im Überstand befindet. Von hier aus kann eine Konzentration z. B. mittels Cross-flow-Filtration oder über Amicon-Kammern vorgenommen werden.

Allerdings hat sich gezeigt, daß ein Teil der in der Enzymzusammensetzung befindlichen Enzyme membrangebunden ist, so daß zur Enzymgewinnung die Zellen vorteilhaft aufgeschlossen werden. Dieser Aufschluß kann z. B. mittels Ultraschall oder einer French-Press erfolgen. Anschließend kann, wie oben beschrieben, eine weitere Konzentration der Enzyme vorgenommen werden.

Charakterisierung der Enzymzusammensetzung

Die erfindungsgemäße Enzymzusammensetzung kann aus einem Enzym oder mehreren Enzymen bestehen. Wird diese Zusammensetzung aus einer den neuen Mikroorganismus enthaltenden Mischkultur gewonnen, können auch Fremdenzyme anderer Mikroorganismen enthalten sein.

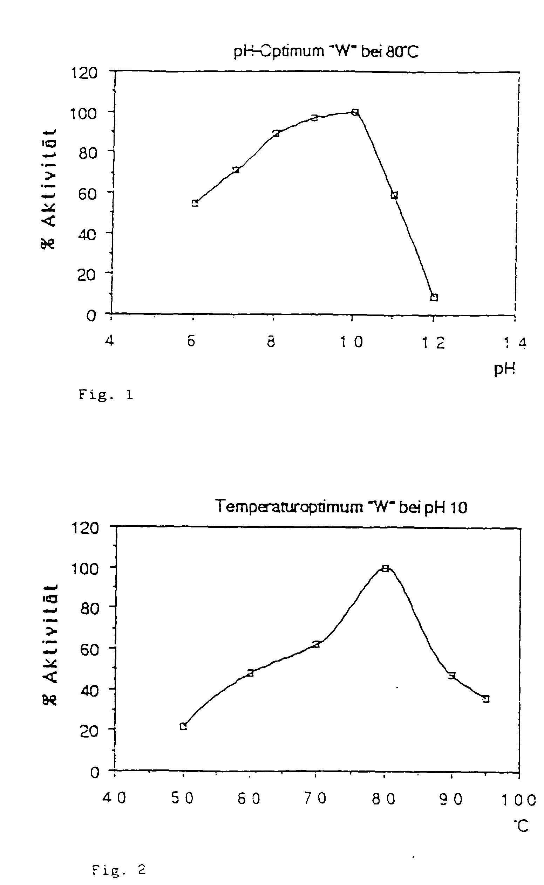

In mehreren Versuchen wurde die Proteaseaktivität der Enzymzusammensetzung bestimmt (Klingeberg M., Haswa F., Antranikian G., (1990), Properties of extremely thermostable protease from anaerobic hyperthermophilic bacteria. Appl. Microbiol. Biotechnol. 43: 715 - 719). Hierbei werden 0,25 %ige Substratlösungen (Casein in B & R-Puffer des gewünschten pH-Wertes) eingesetzt und die Aktivität wie oben angegeben bestimmt. Fig. 1 zeigt das pH-Optimum bei 80°C. Dieses liegt im Bereich von pH 4 bis pH 12, vorzugsweise im Bereich von pH 7 bis pH 11, insbesondere im Bereich von pH 9 bis pH 10. Das Temperaturoptimum gemäß Fig. 2 wurde bei pH 10 ermittelt. Man erkennt eine Enzymaktivität im weiten Bereich von 50 bis 105°C, vorzugsweise im Bereich von 70 bis 90°C, insbesondere bei 80°C. Zur Bestimmung der Thermostabilität werden nach 15', 30', 1 h, stündlich bis 6 h und nach 20 h Proben entnommen und die Aktivität bestimmt. Stabil bedeutet, daß zwischen 6 h und 20 h kein nennenswerter (> 10 %) Aktivitätsverlust eintritt.

Einfluß verschiedener Inhibitoren auf die Proteaseaktivität

Die Hemmwirkung verschiedener Inhibitoren auf die Proteaseaktivität gibt Aufschluß über das aktive Zentrum und den Reaktionsmechanismus der Enzyme, Phenylmethylsulfonylfluorid (PMSF) und Diisopropylfluorophosphat (DFP) binden an die OH-Gruppe des Serins im aktiven Zentrum von Serinproteasen und hemmen letztere irreversibel. Ethylendiamintetraacetat $Na_2$-Salz (EDTA) ist ein Metallchelator und hemmt Metalloproteasen. Proteasen, bei denen Cystein im aktiven Zentrum vorliegt, lassen sich durch Jodacetat irreversibel hemmen. Zur Bestimmung des aktiven Zentrums der thermostabilen Proteasen werden diese zusammen mit entsprechenden Inhibitoren bei Raumtemperatur und pH 7,0 inkubiert, um eine Hydrolyse von DFP und PMSF zu vermeiden.

Reaktionsansatz:

470 µl 0,25 % (w/v) Casein in 50 mM, pH 10 - Puffer (B & R)
25 µl Enzymlösung
5 µl Inhibitorstammlösung (s. u.)

Bei Einsatz von 5 µl Inhibitorstammlösung entspricht die Endkonzentration 1 mM. Bei Einsatz von 25 µl Inhibitorlösung (Endkonzentration 5 mM) werden entsprechend nur 450 µl Substratlösung eingesetzt. Die Ansätze werden zunächst ohne Substrat bei Raumtemperatur (1 h) und anschließend mit Substrat 1 h bei der für das Enzym optimalen Temperatur inkubiert.

Nach der Inkubation wird die Reaktion mit 500 µl einer 10 %igen (w/v) Trichloressigsäurelösung gestoppt und die verbleibende Aktivität, wie oben beschrieben, bestimmt. Folgende Inhibitorlösungen werden eingesetzt:

| PMSF | 17,3 % (w/v) in Ethanol |
| EDTA | 3,56 % (w/v) in $H_2O$ bidest |
| Jodacetat | 14,4 % (w/v) in $H_2O$ bidest |

Hydrolyse chromogener Peptidsubstrate durch Proteasen des Kulturüberstands ($E_{405}$-Wert)

Zur Bestimmung der Substratspezifität der Proteasen werden die genannten chromogenen Peptidsubstrate in einer Endkonzentration von 0,2 mM zusammen mit zellfreiem Rohextrakt inkubiert.

Die Inkubation erfolgt unterhalb der Temperatur- und pH-Optima der Proteasen, um eine thermische bzw. pH-abhängige, nichtenzymatischee Hydrolyse der Peptidsubstrate zu vermeiden.

Reaktionsansatz:

900 µl 0,2 mM (w/v) Substratlösung in 50 mM
Na-P-Puffer, pH 7,0
100 µl Enzymprobe
Inkubation für 17 h bei 60°C

Anschließend wird das freigesetzte p-Nitroanilin sofort gegen einen nicht inkubierten Leerwert bei einer Extinktion von 405 nm gemessen.

SDS-Gelelektrophorese

Das SDS-Gel hat eine Schichtdicke von 1,5 mm. Die Konzentration des Sammelgels beträgt 4,5 % (w/v), die des Trenngels 11,5 % (w/v). Zur Herstellung der Gele werden folgende Lösungen eingesetzt:

| Acrylamidlösung: | |
| --- | --- |
| Acrylamid | 29,2 g |
| N,N-Methylen-Bisacrylamid | 0,8 g |
| $H_2O$ dest. | ad 100 ml |

Zur Entfernung ungelöster Partikel wird diese Lösung filtriert.

| Trenngelpuffer: | |
| --- | --- |
| Tris ($\alpha,\alpha,\alpha$-Tris(hydroxymethyl)-methylamin) | 18,15 g |
| SDS | 0,40 g |
| $H_2O$ dest. | ad 100 ml |

Vor der Zugabe von SDS wird der pH-Wert mit konzentrierter HCl auf 8,9 eingestellt.

| Sammelpuffer: | |
|---|---|
| Tris | 6,05 g |
| SDS | 0,40 g |
| $H_2O$ dest. | ad 100 ml |

Vor der Zugabe von SDS wird der pH-Wert mit konzentrierter HCl auf 6,8 eingestellt.

| Ammoniumpersulfat-Lösung: | |
|---|---|
| Ammoniumpersulfat | 0,10 g |
| $H_2O$ dest. | 0,94 g |

| Elektrophoresepuffer: | |
|---|---|
| Tris | 3,00 g |
| Glycin | 14,40 g |
| SDS | 1,00 g |
| $H_2O$ dest. | ad 1000 ml |

Die Ammoniumpersulfatlösung wird entweder vor Gebrauch frisch angesetzt oder aber portionsweise in Eppendorf-Reaktionsgefäßen bei -20°C gelagert und erst kurz vor Gebrauch aufgetaut. Während der Elektrophoresepuffer stets frisch angesetzt wird, können alle anderen Lösungen mehrere Wochen bei 4°C gelagert werden.

Herstellung der Gele

Zur Herstellung der Plattengele wird zunächst das Trenngel gegossen. Dazu werden

| Trenngelpuffer | 1,4 ml |
|---|---|
| Acrylamid-Lösung | 2,1 ml |
| $H_2O$ dest. | 2,6 ml |

gut vermischt und mit Hilfe einer Wasserstrahlpumpe entgast. Durch Zugabe von

| Ammoniumpersulfatlösung | 40 µl |
|---|---|
| TEMED | 6 µl |

wird die Polymerisation gestartet und die Lösung so weit in die Kammer gefüllt, bis noch 1,5 cm Raum für das Sammelgel verbleibt. Das Überschichten des Gels mit 1 ml $H_2O$ dest. verhindert ein Austrocknen. Nach ca. 60 min ist der Polymerisationsvorgang abgeschlossen, und das Sarnmelgel kann gegossen werden. dazu werden

| Sammelgelpuffer | 0,70 ml |
|---|---|
| Acrylamid-Lösung | 0,26 ml |
| $H_2O$ dest. | 1,04 ml |

vermischt, entgast und die Polymerisation durch Zugabe von

| Ammoniumpersulfatlösung | 10 µl |
|---|---|
| TEMED (N,N,N',N'-Tetramethylethylendiamin) | 2 µl |

gestartet. Vor dem Gießen des Sammelgels wird zunächst das Wasser vom Trenngel dekantiert. Ein luftblasenfrei in das noch flüssige Gel eingesetzter Kamm dient der Taschenformung.

Vorbereitung der Proben

Bis zu 50 µl Proteinlösung mit bis zu 50 µg Protein werden mit 10 µl Denaturierungspuffer versetzt, der folgende Komponenten enthält:

| | |
|---|---|
| SDS | 2,00 g |
| 2-Mercaptoethanol | 3,00 g |
| 10 mM NaP-Puffer, pH 7,0 | ad 100 ml |

Der Ansatz wird 5 min bei 100°C inkubiert, anschließend mit 2 µl Bromphenolblau-Lösung sowie einer Spatelspitze Saccharose versetzt und mit einer Hamilton-Spritze auf das Gel aufgetragen.

Durchführung der Elektrophorese

Zum Einwandern der Proben wird zunächst eine konstante Stromstärke von 10 mA/1,5 mm Geldicke und 10 cm Gelbreite angelegt. Nachdem die Proben in das Sammelgel eingewandert sind, wird die Stromstärke auf 20 mA erhöht.

Silberfärbung

Bei der gelelektrophoretischen Auftrennung sehr geringer Proteinmengen erfolgt die Detektion der Proteinbanden durch Anfärben mit Silber. das Gel wird dazu nach der Elektrophorese in den nachfolgend aufgeführten Lösungen auf einem Schüttler inkubiert

| | |
|---|---|
| Fixierer | 60 min |
| 50 % (v/v) EtOH | $3 \times 20$ min |
| 0,03 % (w/v) $Na_2S_2O_5$ | 1 min |
| $H_2O$ dest. ($3 \times$ gewechselt) | 1 min |
| Silberlösung | 20 min |

und zweimal mit $H_2O$ dest. gespült. Anschließend wird das Gel so lange unter leichtem Schwenken in Entwicklerlösung inkubiert, bis die Proteinbanden deutlich sichtbar werden. Die Färbereaktion wird durch Einlegen des Gels in eine 50 mM EDTA-Lösung gestoppt.

In der letztgenannten Lösung läßt sich das Gel über mehrere Wochen lagern.

| Fixierer: | Ethanol | 30 ml |
|---|---|---|
| | Essigsäure | 10 ml |
| | Formaldehydlösung (37 %) | 50 µl |
| | $H_2O$ dest. | ad 100 ml |

| Silberlösung: | $AgNO_3$ | 0,1 g |
|---|---|---|
| | Formaldehydlösung (37 %) | 75 µl |
| | $H_2O$ dest. | ad 100 ml |

| Entwickler: | $Na_2CO_3$ | 6 g |
|---|---|---|
| | $Na_2S_2O_5$ | 0,4 mg |
| | Formaldehydlösung (37 %) | 50 µl |
| | $H_2O$ dest. | ad 100 ml |

Nachweis proteolytischer Aktivität im SDS-Polyacrylamidgel

Enzyme mit proteolytischer Aktivität können indirekt über die Färbung von Gelatine nachgewiesen werden, die in das Gel eingegossen wird. Dazu wird, wie oben beschrieben, ein 11 %iges SDS-Trenngel gegossen. In Abweichung wird jedoch anstatt 2,6 ml $H_2O$ lediglich 2,0 ml eingesetzt und die restlichen 0,6 ml durch 1 % Gelatine in 100 mM Glycinpuffer, pH 8,5 ersetzt. Dadurch ergibt sich im Gel eine Endkonzentration von 0,1 % Gelatine. Das Sammelgel wird, wie oben beschrieben, ohne Gelatine gegossen. Die Vorbereitung der Proben und die Durchführung der Elektrophorese erfolgt wie oben. An die Elektrophorese schließt sich eine 60minütige Inkubation des Gels in einer 2,5 %igen Triton X-100-Lösung bei 4°C an, um SDS aus dem Gel herauszulösen. Anschließend wird das Gel je nach aufgetragener Aktivität für 5 - 30 min in einem 50 mM Puffer unter optimalen Bedingungen (pH 10, 80°C) inkubiert und anschließend für 60 min in Färbelösung fixiert und gefärbt. Banden mit proteolytischer Aktivität werden nach ca. 3 stündigem Entfärben als helle Banden im blau angefärbten Gel sichtbar.

| Färbelösung: | Amidoschwarz | 0,1 g |
| --- | --- | --- |
| | Ethanol | 30 ml |
| | Eisessig | 10 ml |
| | $H_2O$ dest. | ad 100 ml |

| Entfärber: | Ethanol | 30 ml |
| --- | --- | --- |
| | Eisessig | 10 ml |
| | $H_2O$ dest. | ad 100 ml |

Gelfiltration auf Superdex 200 (HPLC)

<u>Enzymrohextrakt:</u>
1 mg/ml Protein
0,6 U/ml
<u>Säule</u>: Superdex high-load 200 16/60 (120 ml)
<u>Puffer</u>: NaP pH 6,8
<u>Flußrate</u>: 0,5 ml/min
<u>aktiver Proteinpeak:</u> Im Ausschlußvolumen, ist (größtes Eichprotein: 200.000) also größer als 200.000

Einfluß von Metallionen auf die proteolytische Aktivität

| Metallion | ca. % Aktivität 1 mM Ca. | % Aktivität 5 mM |
| --- | --- | --- |
| $AgCl_2$ | 0 | 0 |
| $CaCl_2$ | 85 | 117 |
| $CoCl_2$ | 143 | 187 |
| $CuCl_2$ | 17 | 0 |
| $FeCl_2$ | 127 | 0 |
| $FeCl_3$ | 93 | 0 |
| $MgCl_2$ | 171 | 125 |
| $MnCl_2$ | 110 | 120 |
| NaCl | 86 | 116 |
| $NaMoO_4$ | 91 | 95 |
| $NaSe_2O_3$ | 96 | 136 |
| $NaWO_4$ | 75 | 134 |
| $NiCl_2$ | 72 | 44 |
| $VOSO_4$ | 87 | 44 |
| $ZnCl_2$ | 57 | 37 |

Die Metallionen werden in einem Puffer, 50 mM Na-P; pH 6,8, gelöst. 50 µl dieser Lösung werden mit 50 µl Rohextrakt für 1 h bei Raumtemperatur inkubiert, dann werden 400 µl Substrat (0,25 %, pH 10) zugegeben und für 1 h bei 80°C inkubiert. TCA-Fällung und Aktivitätsbestimmung wird wie oben beschrieben durchgeführt.

In einem weiteren Versuch wurde das Wachstum des neuen Mikroorganismus auf Komplexmedium mit 0,25 % Federn als Substrat untersucht und gleichzeitig die Enzymaktivität ermittelt (Fig. 3). Wird dem Komplexmedium 0,25 % Trypton zugesetzt (Fig. 4), steigt die Enzymaktivität deutlich an.

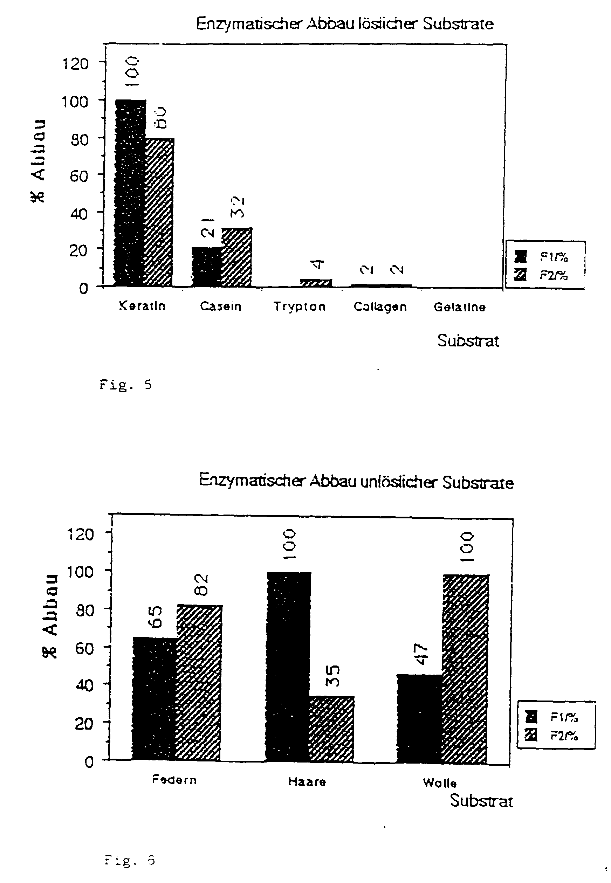

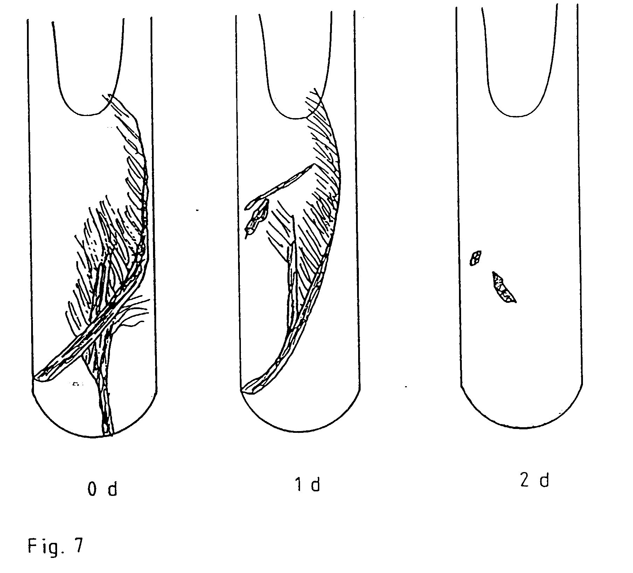

Fig. 5 und 6 zeigen schließlich den Abbau löslicher und unlöslicher Substrate. Nach Zugabe einer definierten Enzymmenge (0,1 U/ml) wurde der prozentuale Substratabbau ermittelt, wobei jeweils auf Federsubstrat gewachsene Zellen des neuen Mikroorganismus (linker Balken, F 1) mit auf Trypton gewachsenen Zellen (rechter Balken, F 2) verglichen wurden.

Ein enzymatischer Proteinabbau kann nach allem mit ganzen Zellen des neuen Mikroorganismus Stamm W durchgeführt werden oder in vitro in Gegenwart einer erfindungsgemäßen Enzymzusammensetzung. Die in-vitro-Behandlung hat hierbei noch die Möglichkeit, daß eine Proteolyse auch unter anaeroben Bedingungen bei 50 bis 100°C und z. B. bei pH 10 durchführbar ist, wobei die anaerobe Variante (s. o.) bevorzugt wird.

**Patentansprüche**

1. Mikroorganismus Stamm W, hinterlegt unter DSM 7003.

2. Enzymzusammensetzung, erhältlich aus einer den Mikroorganismus nach Anspruch 1 beinhaltenden Kultur, durch Kultivieren des Mikroorganismus zur Enzymproduktion oder Enzyminduktion in einem Tryptonmedium und Abtrennung der in Rein- oder Mischkultur angezogenen Zellen aus dem Kulturüberstand bei 10.000 x g und optionell weiterer Konzentration der Enzymzusammensetzung durch Cross-flow-Filtration oder über Amicon-Kammern.

3. Enzymzusammensetzung, erhältlich aus einer den Mikroorganismus nach Anspruch 1 beinhaltenden Kultur, durch Kultivieren des Mikroorganismus zur Enzymproduktion oder Enzyminduktion in einem Tryptonmedium und Aufschluß der in Rein- oder Mischkultur angezogenen Zellen mittels Ultraschall oder einer French-Press und nachfolgende Abtrennung der aufgeschlossenen Zellen und optionelle weitere Konzentration.

4. Enzymzusammensetzung, erhältlich aus einer den Mikroorganismus nach Anspruch 1 aufweisenden Kultur, **dadurch gekennzeichnet,** daß sie ein oder mehrere Proteaseaktivität besitzende Enzyme aufweist, bei dem oder bei denen

   - das Temperaturoptimum (bei pH 10) im Bereich von 70 bis 90 °C, insbesondere bei 80 °C liegt;

   - das pH-Optimum (bei 80 °C) im Bereich von pH 7 bis pH 11, insbesondere im Bereich von pH 9 bis pH 10 liegt;

   - Gelfiltration ein Enzym oder Enzymkomplex mit einem Molekulargewicht von ca. 200 000 Dalton ausweist und eine Molekulargewichtsbestimmung mit einem SDS-Aktivitätsgel zwei Banden im Bereich zwischen ca. 200.000 und ca. 330.000 zeigt;

   - der $E_{405}$-Wert (gegen Blindwert) nach 17 h Inkubation bei 60 °C, 0,2 mM Substrat in Phosphatpuffer pH 7 und 0,3 mg/ml Enzymrohextrakt, Meßstrecke 1 cm bei N-Succinyl-Phe-pNA, N-Succinyl-Ala-Ala-Pro-Phe-pNA und Z-Arg-pNA > 0,5, bei H-Gly-Glu-pNA zwischen 0,3 und 0,5, bei Acetyl-Ala-pNA zwischen 0,1 und 0,3 und bei Benzoyl-DL-Arg-pNA, Z-Gly-Pro-pNA, Benzoyl-Lys-pNA, Z-Arg-pNA und Acetyl-Tyr-pNA unter 0,1 liegt;

   - die folgenden Inhibitoren bei den angegebenen Konzentrationen etwa die folgenden Restaktivitäten ergeben:

| Inhibitor | Restakt. (1 mM) | Restakt. (5 mM) |
|---|---|---|
| ohne | 100 % | 100 % |
| Pefabloc SC (Ser.) | 83 % | - |
| PMSF (Ser.) | 14 % | 10 % |
| EDTA (Metallo.) | 93 % | 85 % |
| Iodacetat (Cyst.) | 83 % | 76 % |

   - die Thermostabilitäten wie folgt sind:

     - bei pH 7 und 8,5 über 20 h bei 70 °C und 80 °C stabil,

     - bei 90 °C nach 4 h weniger als 10 % Restaktivität,

     - bei pH 10, 80 °C und 90 °C nach 15 min Totalverlust der Aktivität,

- bei 70 °C etwa 40 % Restaktivität nach 6 h, nach 20 h Totalverlust.

5. Verwendung des Mikroorganismus nach Anspruch 1 und/oder der Enzymzusammensetzung nach einem der Ansprüche 2 oder 3 zum Abbau von Proteinen insbesondere Keratin bzw. von solchen Stoffen, die diese Proteine beinhalten, insbesondere von Federn, Horn oder Haaren.

6. Verfahren zur enzymatischen Hydrolyse von Proteinen, insbesondere Keratin bzw. von solchen Stoffen, die diese Proteine beinhalten, insbesondere von Federn, Horn oder Haaren,
**dadurch gekennzeichnet,**
daß die Hydrolyse mit einer Enzymzusammensetzung gemäß einem der Ansprüche 2 oder 3 und/oder mit dem Mikroorganismus gemäß Anspruch 1 durchgeführt wird.

7. Verfahren zur Hydrolyse von Peptiden, insbesondere von Keratin oder keratinhaltiger Substanz, wie Federn, Haare oder Horn, in Oligopeptide und/oder Aminosäuren bei einer Temperatur > 50 °C,
**dadurch gekennzeichnet,**
daß die Hydrolyse mit einer Enzymzusammensetzung gemäß einem der Ansprüche 2 oder 3 und/oder mit dem Mikroorganismus gemäß Anspruch 1 durchgeführt wird und daß die Hydrolyse bei einer Temperatur zwischen 50 °C und 105 °C und bei einem pH zwischen 4 und <11 erfolgt, wobei der enzymatische Abbau ein Mehrfaches von einem eventuell begleitenden chemischen Abbau beträgt.

8. Verfahren zur Hydrolyse von Peptiden, insbesondere von Keratin oder keratinhaltiger Substanz, wie Federn, Haare oder Horn, in Oligopeptide und/oder Aminosäuren bei einer Temperatur $\geq$ 70 °C,
**dadurch gekennzeichnet,**
daß die Hydrolyse mit einer Enzymzusammensetzung gemäß einem der Ansprüche 2 oder 3 und/oder mit dem Mikroorganismus gemäß Anspruch 1 durchgeführt wird und daß der Abbau anaerob durchgeführt wird.

9. Verfahren nach Anspruch 7,
**dadurch gekennzeichnet,**
daß die Hydrolyse anaerob durchgeführt wird.

10. Verfahren nach Anspruch 8,
**dadurch gekennzeichnet,**
daß die Hydrolyse bei einem pH zwischen 4 und 12 durchgeführt wird.

11. Verfahren zur Hydrolyse gemäß einem der Ansprüche 7 - 10,
**dadurch gekennzeichnet,**
daß das Keratin oder die keratinhaltige Substanz, insbesondere Federn, vor der Hydrolyse nicht bei einer Temperatur $\geq$ 120 °C vorbehandelt werden.

12. Verfahren zur Hydrolyse gemäß einem der Ansprüche 7 - 11,
**dadurch gekennzeichnet,**
daß das Hydrolysat nach Auflösen von mind. 50 Gew.-% des eingesetzten Substrats mit einer oder mehreren anderen Peptidasen weiterbehandelt wird.

13. Verfahren zur Hydrolyse von Keratin oder keratinhaltiger Substanz, wie Federn, Haare oder Horn, in Oligopeptide und/oder Aminosäuren bei einer Temperatur $\geq$ 50 °C nach einem der Ansprüche 7 - 12,
**dadurch gekennzeichnet,**
daß die Hydrolyse mit einer solchen Enzymmenge, einem solchen pH und einer solchen Temperatur durchgeführt wird, daß ein zugegebenes unlösliches Substrat frühestens nach 3 h aufgelöst ist.

14. Verfahren zur Hydrolyse gemäß Anspruch 13,
**dadurch gekennzeichnet,**
daß die Hydrolyse mit einer solchen Enzymmenge, einem solchen pH und einer solchen Temperatur durchgeführt wird, daß ein zugegebenes unlösliches Substrat nach 24 h zu mindestens 50 Gew.-% aufgelöst ist.

## Claims

1. Strain W microorganism, deposited under DSM 7003.

2. Enzyme composition obtainable from a culture containing the microorganism according to claim 1 by culturing the microorganism for enzyme production or enzyme induction in a tryptone medium and separating the cells cultured in a pure or mixed culture from the culture supernatant at 10000 xg and optionally further concentrating the enzyme composition by cross-flow filtration or through Amicon chambers.

3. Enzyme composition obtainable from a culture containing the microorganism according to claim 1 by culturing the microorganism for enzyme production or enzyme induction in a tryptone medium and disrupting the cells cultured in a pure or mixed culture by means of ultrasound or a French press and subsequently separating the disrupted cells and optionally performing further concentration.

4. Enzyme composition obtainable from a culture containing the microorganism according to claim 1, characterised in that
it contains one or more enzymes exhibiting protease activity, in which

   - the optimum temperature (at pH 10) is in the range from 70 to 90°C, in particular at 80°C;

   - the optimum pH (at 80°C) is in the range from pH 7 to pH 11, in particular in the range from pH 9 to pH 10;

   - gel filtration reveals an enzyme or enzyme complex having a molecular weight of approx. 200000 Dalton and molecular weight determination using an SDS activity gel exhibits two bands in the range between approx. 200000 and approx. 330000;

   - the $E_{405}$ value (against a blank) after 17 hours' incubation at 60°C, 0.2 mM substrate in pH 7 phosphate buffer and 0.3 mg/ml of enzyme crude extract, pathlength 1 cm, is > 0.5 at N-succinyl-Phe-pNA, N-succinyl-Ala-Ala-Pro-Phe-pNA and Z-Arg-pNA, between 0.3 and 0.5 at H-Gly-Glu-pNA, between 0.1 and 0.3 at acetyl-Ala-pNA and below 0.1 at benzoyl-DL-Arg-pNA, Z-Gly-Pro-pNA, benzoyl-Lys-pNA, Z-Arg-pNA and acetyl-Tyr-pNA;

   - the stated concentrations of the following inhibitors result in approximately the following residual activities:

| Inhibitor | Residual activity (1 mM) | Residual activity (5 mM) |
| --- | --- | --- |
| none | 100% | 100% |
| Pefabloc SC (Ser) | 83% | - |
| PMSF (Ser) | 14% | 10% |
| EDTA (metallo.) | 93% | 85% |
| Iodoacetate (Cys) | 83% | 76% |

   - thermostability values are as follows:

       - at pH 7 and 8.5, stable for over 20 h at 70°C and 80°C,

       - at 90°C, residual activity less than 10% after 4 hours,

       - at pH 10, complete loss of activity after 15 minutes at 80°C and 90°C

       - at 70°C, approx. 40% residual activity after 6 hours, complete loss after 20 hours.

5. Use of the microorganism according to claim 1 and/or of the enzyme composition according to one of claims 2 or 3 to degrade proteins, in particular keratin, or those substances containing these proteins, in particular feathers, horn or hair.

6. Process for the enzymatic hydrolysis of proteins, in particular keratin, or those substances containing these proteins, in particular feathers, horn or hair, characterised in that hydrolysis is performed with an enzyme composition according to one of claims 2 or 3 and/or with the microorganism according to claim 1.

7. Process for the hydrolysis of peptides, in particular of keratin or keratin-containing matter, such as feathers, hair or horn, to yield oligopeptides and/or amino acids at a temperature of > 50°C,
characterised in that
hydrolysis is performed with an enzyme composition according to one of claims 2 or 3 and/or with the microorganism according to claim 1 and that hydrolysis proceeds at a temperature of between 50°C and 105°C and at a pH of between 4 and <11, wherein the rate of enzymatic degradation is a multiple of that of any accompanying chemical degradation.

8. Process for the hydrolysis of peptides, in particular of keratin or keratin-containing matter, such as feathers, hair or horn, to yield oligopeptides and/or amino acids at a temperature of ≥ 70°C,
characterised in that
hydrolysis is performed with an enzyme composition according to one of claims 2 or 3 and/or with the microorganism according to claim 1 and that degradation is performed anaerobically.

9. Process according to claim 7,
characterised in that
hydrolysis is performed anaerobically.

10. Process according to claim 8,
characterised in that
hydrolysis is performed at a pH of between 4 and 12.

11. Process for hydrolysis according to one of claims 7 to 10,
characterised in that
the keratin or keratin-containing matter, in particular feathers, are not pretreated at a temperature of ≥ 120°C before hydrolysis.

12. Process for hydrolysis according to one of claims 7 to 11,
characterised in that,
once at least 50 wt.% of the introduced substrate has dissolved, the hydrolysate is further treated with one or more other peptidases.

13. Process for the hydrolysis of keratin or keratin-containing matter, such as feathers, hair or horn, to yield oligopeptides and/or amino acids at a temperature of ≥ 50°C according to one of claims 7 to 12,
characterised in that
hydrolysis is performed with such a quantity of enzyme, at such a pH and at such a temperature that an introduced insoluble substrate has been dissolved at the earliest after 3 hours.

14. Process for hydrolysis according to claim 13,
characterised in that
hydrolysis is performed with such a quantity of enzyme, at such a pH and at such a temperature that an introduced insoluble substrate has been at least 50 wt.% dissolved after 24 hours.

**Revendications**

1. Micro-organisme, Souche W, déposé sous le numéro DSM 7003.

2. Composition enzymatique que l'on obtient à partir d'une culture contenant le micro-organisme selon la revendication 1, en cultivant le micro-organisme pour la production enzymatique ou pour l'induction enzymatique dans un milieu de tryptone et en séparant du résidu de culture à 10.000 x g les cellules que l'on a fait croître dans une culture pure ou mixte, et en soumettant le cas échéant la composition enzymatique à une concentration ultérieure par filtration à courant transversal ou à travers des chambres Amicon.

3. Composition enzymatique que l'on obtient à partir d'une culture contenant le micro-organisme selon la revendication 1, en cultivant le micro-organisme pour la production enzymatique ou pour l'induction enzymatique dans un milieu de tryptone et en désagrégeant les cellules que l'on a fait croître dans une culture pure ou mixte en utilisant des ultrasons ou une presse French et en séparant ultérieurement les cellules désagrégées et en les soumettant à une concentration ultérieure éventuelle.

4. Composition enzymatique que l'on obtient à partir d'une culture présentant le micro-organisme selon la revendication 1,
caractérisée
en ce qu'elle présente une ou plusieurs enzymes possédant une activité de protéase, dans laquelle ou dans lesquelles

- la valeur optimale de température (à un pH de 10) se situe dans le domaine de 70 à 90°C, en particulier à 80°C;

- la valeur optimale du pH (à 80°C) se situe dans le domaine d'un pH de 7 à un pH de 11, en particulier dans le domaine d'un pH de 9 à un pH de 10;

- la filtration sur gel indique une enzyme ou un complexe enzymatique possédant un poids moléculaire d'environ 200.000 daltons, et une détermination du poids moléculaire à l'aide d'un gel d'activité contenant SDS indique deux bandes dans le domaine entre environ 200.000 et environ 330.000;

- la valeur $E_{405}$ (par rapport à la valeur de l'essai à blanc), après incubation pendant 17 heures à 60°C, substrat 0,2 mM dans un tampon de phosphate pH 7 et 0,3 mg/ml d'extrait brut enzymatique, tronçons de mesure de 1 cm, se situe, pour N-succinyl-Phe-pNA, pour N-succinyl-Ala-Ala-Pro-Phe-pNA et pour Z-Arg-pNA > 0,5, pour H-Gly-Glu-pNA entre 0,3 et 0,5, pour acétyl-Ala-pNA entre 0,1 et 0,3 et pour benzoyl-DL-Arg-pNA, pour Z-Gly-Pro-pNA, pour benzoyl-Lys-pNA, pour Z-Arg-pNA et pour acétyl-Tyr-pNA < 0,1;

- les inhibiteurs ci-après, dans les concentrations indiquées, fournissent approximativement les activités résiduelles ci-après:

| Inhibiteur | Activité résiduelle (1 mM) | Activité résiduelle (5 mM) |
|---|---|---|
| Néant | 100 % | 100 % |
| Pefabloc SC (Ser.) | 83 % | - |
| PMSF (Ser.) | 14 % | 10 % |
| EDTA (Metallo.) | 93 % | 85 % |
| Iodoacétate (Cyst.) | 83 % | 76 % |

- les thermostabilités sont les suivantes:

  - à un pH de 7 et de 8,5, pendant 20 heures à 70°C et à 80°C; stabilité,

  - à 90°C après 4 heures: activité résiduelle inférieure à 10%,

  - à un pH de 10, à une température de 80°C et 90°C, après 15 minutes: perte totale de l'activité,

  - à 70°C, environ 40% d'activité résiduelle après 6 heures; après 20 heures: perte totale.

5. Utilisation du micro-organisme selon la revendication 1 et/ou de la composition enzymatique selon l'une quelconque des revendications 2 ou 3 pour la décomposition de protéines, en particulier de la kératine, respectivement de substances qui contiennent ces protéines, en particulier de plumes, de corne ou de cheveux.

6. Procédé pour l'hydrolyse enzymatique de protéines, en particulier de la kératine, respectivement de substances qui contiennent ces protéines, en particulier de plumes, de corne ou de cheveux,
caractérisé
en ce qu'on effectue l'hydrolyse avec une composition enzymatique selon l'une quelconque des revendications 2 ou 3 et/ou avec le micro-organisme selon la revendication 1.

7. Procédé pour l'hydrolyse de peptides, en particulier de la kératine ou d'une substance contenant de la kératine, telle que des plumes, des cheveux ou de la corne, pour obtenir des oligopeptides et/ou des acides aminés à une température > 50°C,
caractérisé
en ce qu'on effectue l'hydrolyse avec une composition enzymatique selon l'une quelconque des revendications 2 ou 3 et/ou avec le micro-organisme selon la revendication 1, et en ce que l'hydrolyse a lieu à une température entre 50°C et 105°C et à un pH entre 4 et < 11, la décomposition enzymatique représentant un multiple d'une décomposition chimique éventuellement secondaire.

8. Procédé pour l'hydrolyse de peptides, en particulier de la kératine ou d'une substance contenant de la kératine, telle que des plumes, des cheveux ou de la corne, pour obtenir des oligopeptides et/ou des acides aminés à une température ≥ 70°C,
caractérisé
en ce qu'on effectue l'hydrolyse avec une composition enzymatique selon l'une quelconque des revendications 2 ou 3 et/ou avec le micro-organisme selon la revendication 1, et en ce qu'on effectue la décomposition dans des conditions anaérobies.

9. Procédé selon la revendication 7,
caractérisé
en ce qu'on effectue l'hydrolyse dans des conditions anaérobies.

10. Procédé selon la revendication 8,
caractérisé
en ce qu'on effectue l'hydrolyse à un pH entre 4 et 12.

11. Procédé pour l'hydrolyse selon l'une quelconque des revendications 7 à 10,
caractérisé
en ce qu'on ne soumet pas la kératine ou la substance contenant de la kératine, en particulier des plumes, avant l'hydrolyse, à un prétraitement à une température ≥ 120°C.

12. Procédé pour l'hydrolyse selon l'une quelconque des revendications 7 à 11,
caractérisé
en ce qu'on soumet l'hydrolysat après dissolution d'au moins 50% en poids du substrat mis en oeuvre, à un traitement ultérieur avec une ou plusieurs autres peptidases.

13. Procédé pour l'hydrolyse de la kératine ou d'une substance contenant de la kératine, telle que des plumes, des cheveux ou de la corne, pour obtenir des oligopeptides et/ou des acides aminés à une température ≥ 50°C selon l'une quelconque des revendications 7 à 12,
caractérisé
en ce qu'on effectue l'hydrolyse avec une quantité enzymatique telle, à un pH tel et à une température telle qu'un substrat insoluble ajouté est dissous au plus tôt après 3 heures.

14. Procédé pour l'hydrolyse selon la revendication 13,
caractérisé
en ce qu'on effectue l'hydrolyse avec une quantité enzymatique telle, à un pH tel et à une température telle qu'un substrat insoluble ajouté est dissous après 24 heures à concurrence d'au moins 50% en poids.

pH-Optimum "W" bei 80°C

Fig. 1

Temperaturoptimum "W" bei pH 10

Fig. 2

Wachstum und Enzymaktivität von "WD"

Fig. 3

Wachstum und Enzymaktivität von "WD"

Fig. 4

## Enzymatischer Abbau löslicher Substrate

Fig. 5

## Enzymatischer Abbau unlöslicher Substrate

Fig. 6

0 d          1 d          2 d

Fig. 7